# DEMANDE DE BREVET EUROPEEN

(11) **EP 1 378 512 A1**
(43) Date de publication de la demande: **07.01.2004**
(21) Numéro de dépôt: 03077049.9
(22) Date de dépôt: 07.08.1998
(51) Int. Cl.: C07D 491/04, C07D 491/22, A61K 31/435

(54) **Analogues optiquement purs de la camptothécine**

(30) Priorité: 29.08.1997 FR 9710785
(62) Demande divisionnaire de: 98941567.4
(71) Demandeur: Société de Conseils de Recherches et d'Applications Scientifiques ( S.C.R.A.S.), 75016 Paris (FR)
(72) Inventeur: Cazaux, Jean-Bernard, 30390 Aramon (FR); Lavergne, Olivier, 91120 Palaiseau (FR); Le Breton, Christine, 84000 Avignon (FR); Manginot, Eric, 84140 Montfavet (FR); Bigg, Dennis, 91190 Gif-sur-Yvette (FR)
(74) Mandataire: Bourgouin, André

(57) **Abrégé**

L'invention concerne de nouveaux analogues de la camptothécine et en particulier les produits répondant aux formules suivantes :
- (+)-5-éthyl-9,10-difluoro-5-hydroxy-4,5,13,15-tétrahydro-1H, 3H-oxépino[3',4':6,7]indolizino[1,2-b]quinoline-3,15-dione ;
- (+)-chlorure de 1-[9-chloro-5-éthyl-5-hydroxy-10-méthyl-3,15-dioxo-4,5,13,15-tétrahydo-1H, 3H-oxépino[3',4':6,7]indolizino[1,2-b]quinolin-12-ylméthyl]-4-méthyl-hexahydropyridinium ;
leur application comme médicaments, les compositions pharmaceutiques les renfermant et leur utilisation pour la réalisation de médicaments antitumoraux, antiviraux ou antiparasitaires.

Elle concerne également un nouvel intermédiaire dans la synthèse des produits susmentionnés ainsi qu'un procédé de préparation dudit intermédiaire.

## Description

La camptothécine est un composé naturel qui a été isolé pour la première fois des feuilles et de l'écorce de la plante chinoise appelée *camptotheca acuminata* (voir Wall et ses collaborateurs, J. Amer. Chem. Soc. 88:3888 (1966)). La camptothécine est un composé pentacyclique constitué d'un fragment indolizino[1,2-b]quinoléine fusionné avec une α-hydroxylactone à six chaînons. Le carbone en position 20, qui porte le groupe α-hydroxy, est asymétrique et confère à la molécule un pouvoir rotatoire. La forme naturelle de la camptothécine possède la configuration absolue "S" au carbone 20 et répond à la formule suivante :

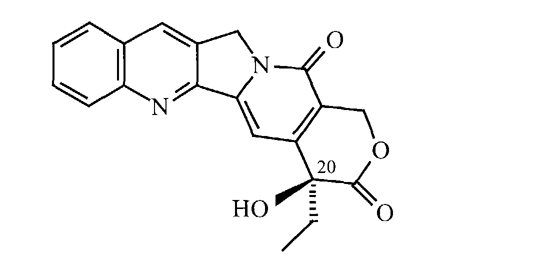

La camptothécine présente une activité anti-proliférative dans plusieurs lignées cellulaires cancéreuses, comprenant les lignées cellulaires de tumeurs humaines du colon, du poumon et du sein (Suffness, M. et collaborateurs : The Alkaloids Chemistry and Pharmacology, Bross, A., ed., Vol. 25, p. 73 (Academic Press, 1985)). On suggère que l'activité anti-proliférative de la camptothécine est en relation avec son activité inhibitrice de la topoisomérase I de l'ADN.

Il a été indiqué que l'α-hydroxylactone était une exigence absolue à la fois pour l'activité *in vivo* et *in vitro* de la camptothécine (Camptothecins : New Anticancer Agents, Putmesil, M., et ses collaborateurs, ed., p. 27 (CRC Press, 1995); Wall, M. et ses collaborateurs, Cancer Res. 55:753 (1995); Hertzberg et ses collaborateurs, J. Med. Chem. 32:715 (1982) et Crow et ses collaborateurs, J. Med. Chem. 35:4160 (1992)). Plus récemment, la demanderesse a mis au point une nouvelle classe d'analogues de la camptothécine, dans lesquels une β-hydroxylactone remplace l'α-hydroxylactone naturelle de la camptothécine (cf. demande de brevet WO 97/00876).

L'invention a pour objet un nouveau procédé de préparation d'un intermédiaire de synthèse énantiomériquement pur, ainsi que de nouveaux analogues énantiomériquement purs de la camptothécine.

L'invention a donc d'abord pour objet de nouveaux analogues de la camptothécine qui diffèrent de tout composé connu, caractérisés en ce qu'il ont les formules respectives (I) et (II) représentées ci-dessous 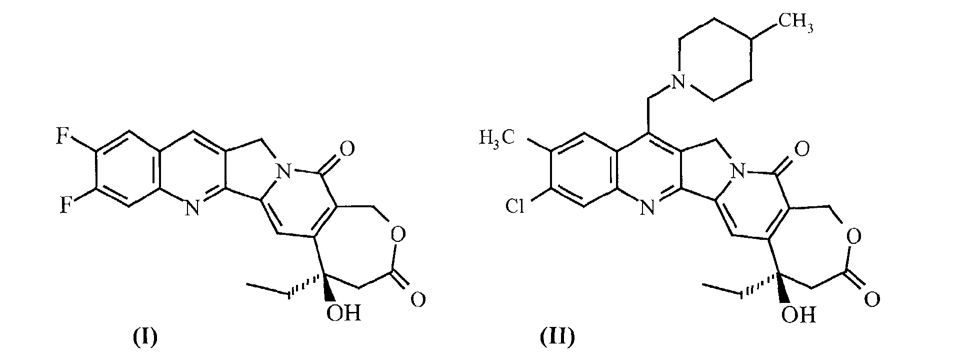 ou en ce qu'il s'agit de sels du composé de formule (**II**) tel par exemple celui de formule (**III**) représentée ci-dessous

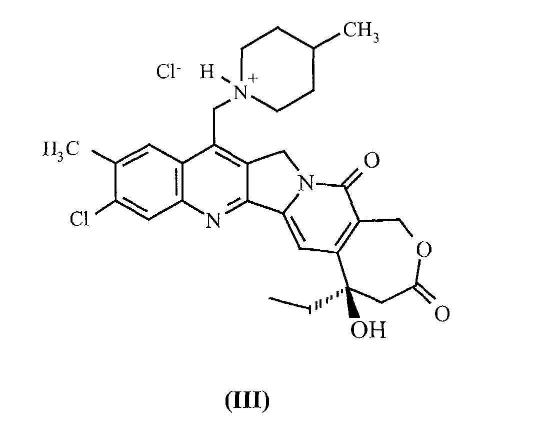

Un intermédiaire-clé dans la synthèse de ce type de composés optiquement purs est un produit de formule générale **M** représentée ci-dessus 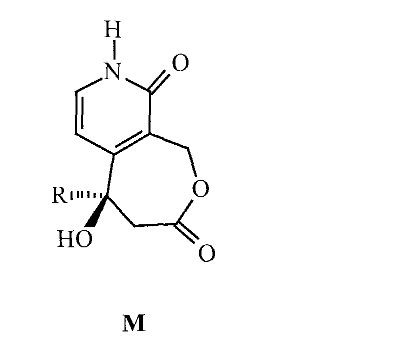 dans laquelle R représente un radical alkyle linéaire ou ramifié comptant de 1 à 10 atomes de carbone. De préférence, R représente un radical éthyle.

Les composés de formules **(I)** et **(II)** peuvent être préparés de la façon suivante :
- on couple le composé de formule 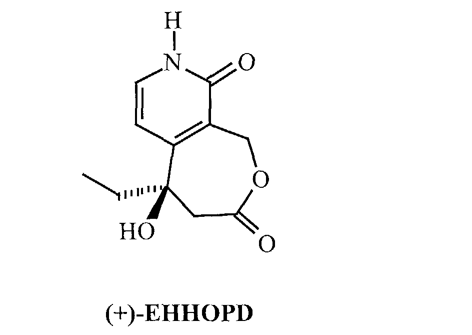 avec respectivement l'un ou l'autre des composés de formules **N**_{**1**} ou **N**_{**2**} représentées ci-dessous: 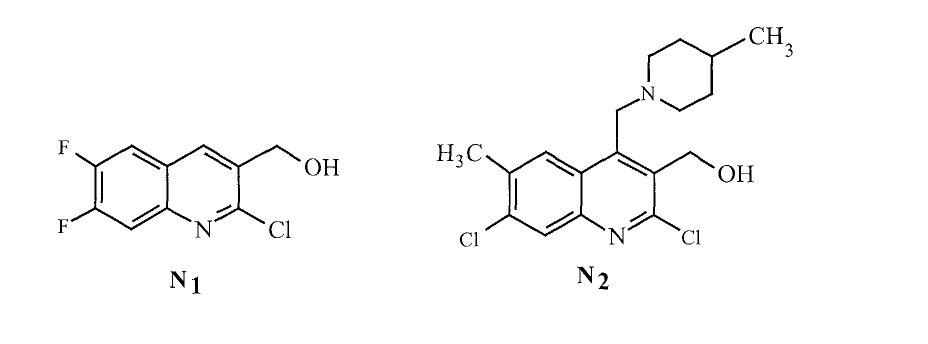 pour donner respectivement le composé de formule **O**_{**1**} ou le composé de formule **O**_{**2**}: 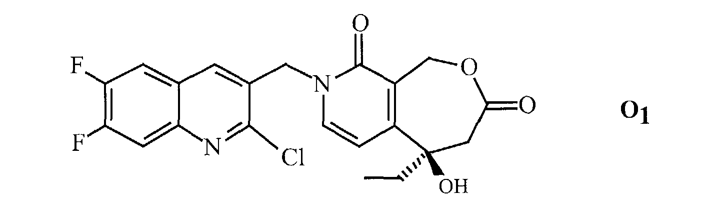 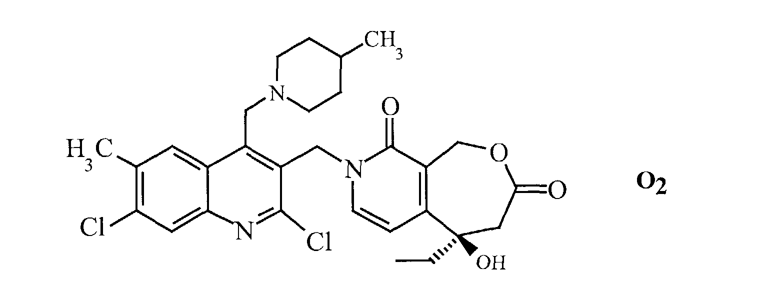
- le composé **O**_{**1**} est ensuite cyclisé, et on obtient alors le composé de formule **(I);** la cyclisation du composé **O**_{**2**} donne le composé de formule **(II),** lequel peut, après salification, donner le composé de formule **(III).**

La formation des composés **O**_{**1**} ou **O**_{**2**} à partir du composé de formule générale **M** pour lequel R représente un radical éthyle et **N**_{**1**} ou **N**_{**2**} s'effectue par un traitement connu de l'homme de l'art sous le nom de réaction de Mitsunobu (se référer à Mitsunobu, O. et coll. *Synthesis,* p.1 (1981)). Il s'agit de déplacer la fonction hydroxyle du composé N par un nucléophile tel que le composé M, ou un dérivé déprotoné de ce dernier, par un traitement avec une phosphine, par exemple la triphénylphosphine, et un dérivé azodicarboxylate, par exemple l'azodicarboxylate de diéthyle ou de diisopropyle, dans un solvant aprotique tel que, par exemple, le tétrahydrofurane ou le *N,N*-diméthylformamide. La cyclisation des composés **O**_{**1**} et **O**_{**2**} pour donner les composés de formules **(I)** et **(II)** s'effectue de préférence en présence d'un catalyseur palladié (par exemple le diacétate de palladium) dans des conditions basiques (fournies par exemple par un acétate alcalin éventuellement combiné à un agent de transfert de phase tel que par exemple le bromure de tétrabutylammonium), dans un solvant aprotique tel que l'acétonitrile ou le *N,N*-diméthylformamide, à une température comprise entre 50° C et 120° C (R. Grigg et coll., *Tetrahedron* 46, page 4003 (1990)).

L'invention offre également, à titre de produit industriel nouveau, un composé de formule générale **M** tel que défini précédemment. Ce produit peut être utilisé pour la fabrication de médicaments.

Le composé de formule **M** est synthétisé selon un procédé nouveau faisant partie de l'invention et constitué des étapes successives suivantes :
- l'ester *t*-butylique racémique représenté ci-dessous 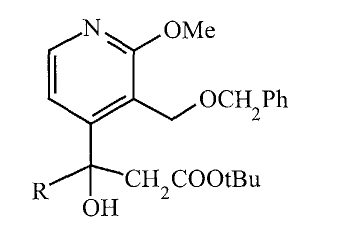 (pour sa préparation, se référer notamment à la demande de brevet WO 97/00876) est traité par de l'acide trifluoroacétique pendant 18 h à température ambiante pour donner l'acide carboxylique correspondant ;
- on chauffe ensuite dans de l'alcool isopropylique le sel de quinidine de l'acide obtenu précédemment à une température supérieure à 30 °C, et de préférence de 50 °C environ, avant de laisser refroidir le milieu réactionnel jusqu'à température ambiante, de sorte que le sel d'un des énantiomères de l'acide susmentionné cristallise tandis que le sel de l'autre énantiomère, dont l'anion est représenté ci-dessous, reste en solution 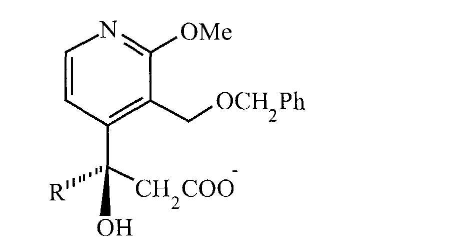
- la solution dans l'alcool isopropylique du sel de l'énantiomère n'ayant pas cristallisé est concentrée et traitée à l'acide chlorhydrique et agitée, donnant le composé de formule générale **A** représentée ci-dessous 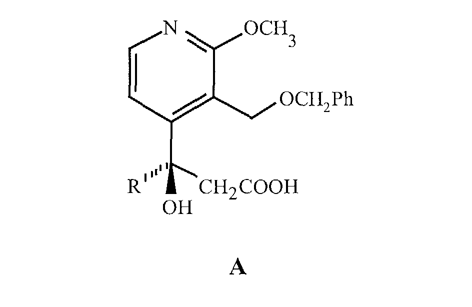
- le composé de formule générale **A** est ensuite mis en contact avec du palladium sur charbon humide, puis on ajoute du formiate d'ammonium ou de l'acide formique au mélange pour donner le produit débenzylé de formule générale **B** représenté ci-dessous 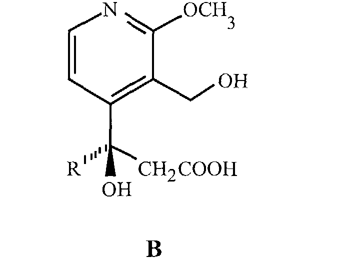
- on cyclise ensuite le composé de formule générale **B** par action de dicyclohexylcarbodiimide pour obtenir le composé lactonique de formule générale **C** représentée ci-dessous 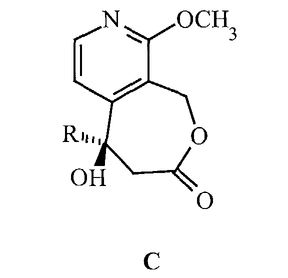
- enfin, on transforme le groupement -OCH₃ du composé lactonique de formule générale **C** en carbonyle, par action d'iodure de sodium et de chlorure de triméthylsilyle, pour obtenir un composé de formule générale **M** représentée ci-dessous.

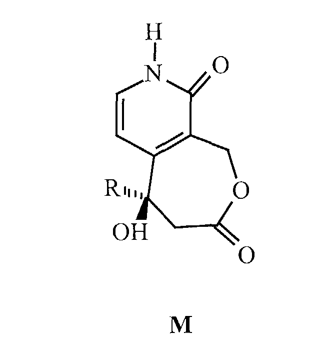

Pour le procédé décrit ci-dessus, la réaction conduisant du composé de formule générale **A** au composé de formule générale **B** se fera de préférence dans du méthanol, et de préférence en chauffant le milieu réactionnel à 40°C environ après l'addition de formiate d'ammonium. La cyclisation du composé de formule générale **B** pour donner le composé **C** pourra s'effectuer dans du THF, de préférence à une température de 50 °C environ, tandis qu'on travaillera de préférence à température ambiante avec l'acétonitrile comme solvant dans la réaction conduisant du composé de formule générale **C** au composé de formule générale **M**.

Dans le cas particulier où R représente un groupe éthyle, le composé de formule **M** est synthétisé selon le procédé constitué des étapes successives suivantes :
- l'ester *t*-butylique racémique représenté ci-dessous 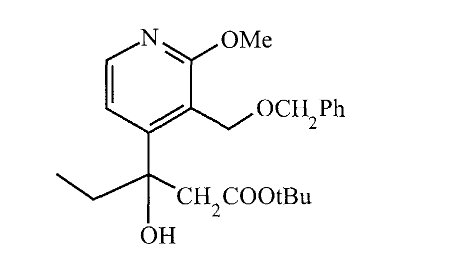 (pour sa préparation, se référer notamment à la demande de brevet WO 97/00876) est traité par de l'acide trifluoroacétique durant 18 h à température ambiante pour donner l'acide carboxylique correspondant ;
- on chauffe dans de l'alcool isopropylique le sel de quinidine de l'acide 3-(3-benzyloxyméthyl-2-méthoxy-4-pyridyl)-3-hydroxy-pentanoïque à une température supérieure à 30 °C, et de préférence de 50 °C environ, avant de laisser refroidir le milieu réactionnel jusqu'à température ambiante, de sorte que le sel de l'énantiomère (+) de l'acide 3-(3-benzyloxyméthyl-2-méthoxy-4-pyridyl)-3-hydroxy-pentanoïque cristallise tandis que le sel de l'isomère (-), dont l'anion est représenté ci-dessous, reste en solution 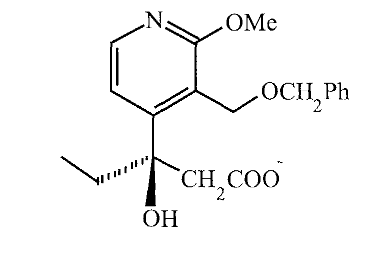
- la solution dans l'alcool isopropylique du sel de l'énantiomère (-) de l'acide 3-(3-benzyloxyméthyl-2-méthoxy-4-pyridyl)-3-hydroxy-pentanoïque est concentrée et traitée à l'acide chlorhydrique et agitée, donnant le composé de formule **A'** représentée ci-dessous 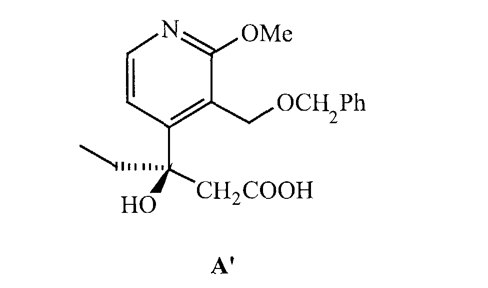
- le composé **A'** est ensuite mis en contact avec du palladium sur charbon humide, puis on ajoute du formiate d'ammonium ou de l'acide formique au mélange pour donner le produit débenzylé **B'** représenté ci-dessous 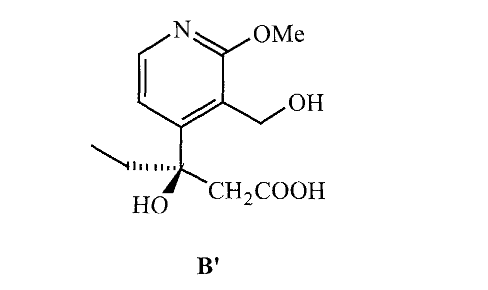
- on cyclise ensuite le composé de formule **B'** par action de dicyclohexylcarbodiimide pour obtenir le composé lactonique de formule **C'** représentée ci-dessous 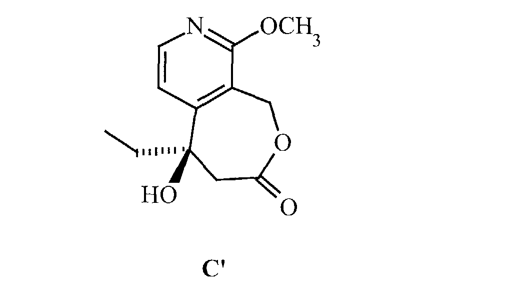
- enfin, on transforme le groupement -OCH₃ du composé lactonique de formule **C'** en carbonyle, par action d'iodure de sodium et de chlorure de triméthylsilyle, pour obtenir la (+)-5-éthyl-5-hydroxy-1,3,4,5,8,9-hexahydrooxépino[3,4-c]pyridin-3,9-dione (ou (+)-**EHHOPD**) représenté ci-dessous. 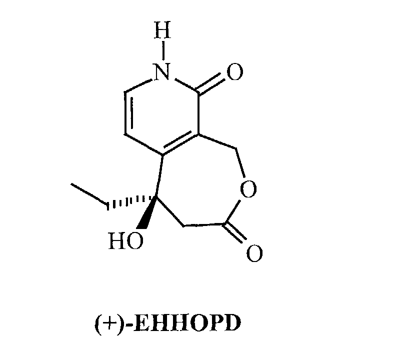 Le composé de formule **N**_{**1**} peut être obtenu à partir de l'aniline de formule **P**_{**1**} représentée ci-dessous 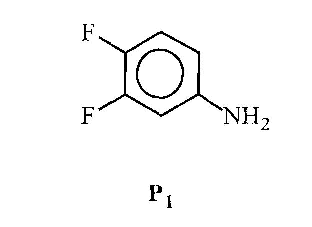 selon le procédé suivant : l'aniline de formule **P**_{**1**} est *N*-acétylée par traitement avec un agent acétylant tel que par exemple l'anhydride acétique. L'acétanilide ainsi obtenu est traité à une température comprise entre 50° C et 100° C, de préférence 75° C, par un réactif connu de l'homme de l'art sous de nom de réactif de Vilsmeyer (obtenu par l'action de l'oxychlorure de phosphoryle sur la *N,N*-diméthylformamide à une température comprise entre 0° C et 10° C) pour donner le 2-chloro-3-quinoléinecarbaldéhyde correspondant (se référer, par exemple à Meth-Cohn, et coll. *J*. *Chem. Soc., Perkin Trans. I* p.1520 (1981); Meth-Cohn, et coll. *J*. *Chem. Soc., Perkin Trans. I* p.2509 (1981); et Nakasimhan et coll. *J. Am. Chem. Soc., 112,* p.4431 (1990)). Le 2-chloro-6,7-difluoro-3-quinoléinecarbaldéhyde est facilement réduit en le 2-chloro-6,7-difluoro-3-quinoléineméthanol correspondant de formule **N**_{**1**}, dans des conditions classiques connues de l'homme de l'art telles que le traitement dans un solvant alcoolique (par exemple le méthanol) par du borohydrure de sodium à une température comprise entre 0° C et 40° C.

Le composé de formule **N**_{**2**} peut être obtenu selon le procédé suivant : l'aniline de formule **P**_{**2**} représentée ci-dessous 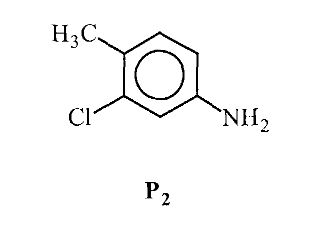 est ortho-acylée par réaction avec le chloroacétonitrile en présence de trichlorure de bore et d'un autre acide de Lewis tel que le trichlorure d'aluminium, le tétrachlorure de titane ou le chlorure de diéthylaluminium dans un solvant aprotique ou un mélange de solvant aprotique, suivie d'une hydrolyse *(cf.* Sugasawa, T, et coll. *J*. *Am. Chem. Soc. 100,* p. 4842 (1978)). L'intermédiaire ainsi obtenu est ensuite traité par le chlorure d'éthylmalonyle dans un solvant aprotique tel que l'acétonitrile en présence d'une base telle que la triéthylamine, puis traité par un alcoolate alcalin, par exemple le méthylate de sodium dans le méthanol, pour donner le 7-chloro-4-chlorométhyl-6-méthyl-2-oxo-1,2-dihydro-3-quinolinecarboxylate d'éthyle. Ce dernier est transformé en 2,7-dichloro-4-chlorométhyl-6-méthyl-3-quinolinecarboxylate d'éthyle par un traitement avec de l'oxychlorure de phosphoryle. On effectue ensuite une substitution nucléophile par traitement avec la 4-méthylpipéridine. La fonction carboxylate d'éthyle est ensuite réduite par l'hydrure de diisobutylaluminium dans un solvant aprotique tel que le dichlorométhane pour donner le composé de formule **N**_{**2**}**.** On peut éventuellement inverser l'ordre des deux dernières étapes.

Des analogues des composés intermédiaires du type de **N**_{**1**} ou **N**_{**2**} ont été décrits dans la littérature et en particulier dans la demande PCT 95/05427.

Le composé de formule (**II**) peut être transformé en sel pharmaceutiquement acceptable selon les méthodes usuelles. Des sels acceptables comprennent, à titre d'exemple et de façon non limitative, des sels d'addition d'acides inorganiques tels que chlorhydrate, sulfate, phosphate, diphosphate, bromhydrate et nitrate ou d'acides organiques tels que acétate, maléate, fumarate, tartrate, succinate, citrate, lactate, méthanesulfonate, p-toluènesulfonate, pamoate, salicylate, oxalate et stéarate. Pour d'autres exemples de sels pharmaceutiquement acceptables, on peut se référer à "Pharmaceutical Salts", J. Pharm. Sci. 66:1 (1977).

Les composés de la présente invention possèdent d'intéressantes propriétés pharmacologiques. C'est ainsi que les composés de la présente invention ont une activité inhibitrice de la topoisomérase I et/ou II et une activité anti-tumorale. L'état de la technique suggère que les composés de l'invention présentent une activité anti-parasitaire et/ou anti-virale. Les composés de la présente invention peuvent ainsi être utilisés dans différentes applications thérapeutiques.

On trouvera ci-après, dans la partie expérimentale, une illustration des propriétés pharmacologiques des composés de l'invention.

Les composés peuvent inhiber la topoisomérase, par exemple de type I et/ou II, chez un patient, par exemple un mammifère tel que l'homme, par administration à ce patient d'une quantité thérapeutiquement efficace d'un composé de formule (**I**) ou de formule (**II**), ou d'un sel pharmaceutiquement acceptable d'un composé de formule (**II**), ou encore d'un mélange quelconque de ces derniers.

Les composés de l'invention possèdent une activité anti-tumorale. Ils peuvent être utilisés pour le traitement des tumeurs, par exemple des tumeurs exprimant une topoisomérase, chez un patient par administration audit patient d'une quantité thérapeutiquement efficace d'un composé de formule (**I**) ou de formule (**II**), ou d'un sel pharmaceutiquement acceptable d'un composé de formule (**II**), ou encore d'un mélange quelconque de ces derniers. Des exemples de tumeurs ou de cancers comprennent les cancers de l'oesophage, de l'estomac, des intestins, du rectum, de 1a cavité orale, du pharynx, du larynx, du poumon, du colon, du sein, du cervix uteri, du corpus endométrium, des ovaires, de la prostate, des testicules, de la vessie, des reins, du foie, du pancréas, des os, des tissus conjonctifs, de la peau, des yeux, du cerveau et du système nerveux central, ainsi que le cancer de la thyroïde, la leucémie, la maladie de Hodgkin, les lymphomes autres que ceux de Hodgkin, les myélomes multiples et autres.

Ils peuvent également être utilisés pour le traitement des infections parasitiques par l'inhibition des hémoflagellates (par exemple dans la trypanosomie ou les infections leishmania) ou par inhibition de la plasmodie (comme par exemple dans la malaria), mais aussi le traitement des infections ou maladies virales.

Ces propriétés rendent les produits de formules (**I**) et (**II**) aptes à une utilisation pharmaceutique. La présente demande a également pour objet, à titre de médicaments, les produits de formules (**I**) et (**II**) telles que définies ci-dessus, ou les sels d'addition avec les acides minéraux ou organiques pharmaceutiquement acceptables du produit de formule (**II**) tel que par exemple le sel de formule (**III**) précédemment décrit, ou encore un mélange quelconque de ces derniers. L'invention concerne de même les compositions pharmaceutiques contenant, à titre de principe actif, l'un au moins des médicaments tels que définis ci-dessus.

L'invention concerne ainsi des compositions pharmaceutiques contenant un composé de l'invention ou un sel additif d'acide pharmaceutiquement acceptable de celui-ci, en association avec un support pharmaceutiquement acceptable suivant le mode d'administration choisie (par exemple orale, intraveineuse, intrapéritonéales, intramusculaires, trans-dermique ou sous-cutanée). La composition pharmaceutique (par exemple thérapeutique) peut être sous forme solide, liquide, de liposomes ou de micelles lipidiques.

La composition pharmaceutique peut être sous forme solide comme, par exemple, les poudres, pilules, granules, comprimés, liposomes, gélules ou suppositoires. La pilule, le comprimé ou la gélule peuvent être revêtus d'une substance capable de protéger la composition de l'action de l'acide gastrique ou des enzymes dans l'estomac du sujet pendant une période de temps suffisante pour permettre à cette composition de passer non digérée dans l'intestin grêle de ce dernier. Le composé peut aussi être administré localement, par exemple à l'emplacement même de la tumeur. Le composé peut aussi être administré selon le processus de la libération prolongée (par exemple une composition à libération prolongée ou une pompe d'infusion). Les supports solides appropriés peuvent être, par exemple, le phosphate de calcium, le stéarate de magnésium, le carbonate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire. Les compositions pharmaceutiques contenant un composé de l'invention peuvent donc également se présenter sous forme liquide comme, par exemple, des solutions, des émulsions, des suspensions ou une formulation à libération prolongée. Les supports liquides appropriés peuvent être, par exemple, l'eau, les solvants organiques tels que le glycérol ou les glycols tel que le polyéthylène glycol, de même que leurs mélanges, dans des proportions variées, dans l'eau.

L'invention a également pour objet l'utilisation des produits de formules (**I**) et (**II**) telles que définies ci-dessus, ou des sels d'addition avec les acides minéraux ou organiques pharmaceutiquement acceptables du produit de formule (**II**), tel que par exemple le sel de formule (**III**) précédemment décrit, ou encore d'un mélange de ces derniers, pour la préparation de médicaments destinés à inhiber les topoisomérases, et plus particulièrement les topoisomérases de type I ou de type II, de médicaments destinés à traiter les tumeurs, de médicaments destinés à traiter les infections parasitaires, ainsi que de médicaments destinés traiter des infections ou maladies virales.

La dose d'un composé selon la présente invention, à prévoir pour le traitement des maladies ou troubles mentionnés ci-dessus, varie suivant le mode d'administration, l'âge et le poids corporel du sujet à traiter ainsi que l'état de ce dernier, et il en sera décidé en définitive par le médecin ou le vétérinaire traitant. Une telle quantité déterminée par le médecin ou le vétérinaire traitant est appelée ici "quantité thérapeutiquement efficace".

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnés ici sont incorporés par référence.

Les exemples suivants sont présentés pour illustrer les procédures ci-dessus et ne doivent en aucun cas être considérés comme une limite à la portée de l'invention.

### PARTIE EXPÉRIMENTALE :

### Exemple 1: (+)-5-Ethyl-5-hydroxy-1,3,4,5,8,9-hexahydrooxépino[3,4-c]pyridin-3,9-dione [(+)-EHHOPD]

### 1.a. Sel de quinidine de l'acide 3-(3-benzyloxyméthyl-2-méthoxy-4-pyridyl)-3-hydroxy-pentanoïque:

Du 3-(3-benzyloxyméthyl-2-méthoxy-4-pyridyl)-3-hydroxy-pentanoate de tertiobutyle (40 g ; 100 mmol) est traité par de l'acide trifluoroacétique (150 ml) et le milieu réactionnel est agité pendant 18 h à 20 °C. Après évaporation de l'acide trifluoroacétique, du chlorure de méthylène (200 ml) est coulé et une solution saturée en bicarbonate de sodium est introduite jusqu'à pH = 7,5-8. Après décantation, la phase aqueuse est lavée avec 100 ml de chlorure de méthylène. Le pH de la phase aqueuse est alors ramené à 1 par ajout d'une solution d'acide chlorhydrique 6 N. Le produit est ensuite extrait de la phase aqueuse par du chlorure de méthylène (2 fois 200 ml). La solution est séchée sur du sulfate de magnésium et concentrée. L'acide 3-(3-benzyloxyméthyl-2-méthoxy-4-pyridyl)-3-hydroxy-pentanoïque (31,1 g ; 90 mmol) ainsi obtenu, repris dans de l'alcool isopropylique (30 ml), est traité par une solution de quinidine (29,2 g ; 90 mmol) dans de l'alcool isopropylique (30 ml) à 50 °C sous agitation jusqu'à dissolution complète. On laisse alors la température redescendre jusqu'à 40 °C, l'agitation est arrêtée et on laisse évoluer la température jusqu'à 20 °C. Le milieu est amené à 0 °C sans agitation puis maintenu à cette température pendant 16 heures. On laisse ensuite la température remonter jusqu'à 20 °C et on agite jusqu'à cristallisation. Le milieu est dilué par de l'alcool isopropylique puis filtré. Le précipité est rincé par de l'alcool isopropylique. Le sel de l'énantiomère (+) précipite (m=26,6 g) alors que le sel de l'énantiomère (-) reste en solution dans l'alcool isopropylique. On recueille donc le filtrat qui est concentré pour donner une huile (34 g) qui est engagée sans autre purification dans l'étape suivante.

Les produits sont analysés par HPLC sur colonne CHIRAL AGP 5µ (10 cm x 4mm) éluée par un mélange alcool isopropylique/eau/tampon phosphate, pH = 6,5 : 30/920/50, à un débit de 1,2 ml/min, détection UV à 280 nm. Les temps de rétention obtenus sont 6,4 minutes pour l'énantiomère (-) et 2,8 minutes pour l'énantiomère (+). Le rapport énantiomère (-) / énantiomère (+) est de 83 / 17.

### 1.b. Acide (-)-3-(3-benzyloxyméthyl-2-méthoxy-4-pyridyl)-3-hydroxy-pentanoïque

La solution dans l'alcool isopropylique du sel de quinidine de l'énantiomère (-) de l'acide 3-(3-benzyloxyméthyl-2-méthoxy-4-pyridyl)-3-hydroxy-pentanoïque (étape 1.a) est concentrée. Le concentrat est repris dans 270 ml de chlorure de méthylène et 270 ml d'une solution d'acide chlorhydrique 1N. Le milieu réactionnel est agité pendant 16 heures à 20°C. Après décantation, la phase organique est concentrée, le concentrat est repris dans le méthanol pour être engagé dans la phase suivante.

On obtient 13,5 g de produit (rendement de 87%) et une proportion énantiomère (-) / énantiomère (+) de 85 / 15.

Les temps de rétention HPLC (même protocole qu'en 1.a.) sont :
- énantiomère (-) : 6,4 minutes
- énantiomère (+) : 2,8 minutes

### 1.c. (+)-5-Ethyl-5-hydroxy-1,3,4,5,8,9-hexahydrooxépino[3,4-c] pyridin-3,9-dione :

L'acide (-)-3-(3-benzyloxyméthyl-2-méthoxy-4-pyridyl)-3-hydroxy-pentanoïque (13,5 g ; 39 mmol ; étape 1.b) est mis en solution dans 87 ml de méthanol. Cette solution est coulée sous azote sur du Palladium 10% sur charbon humide à 50% (27,7 g ; 13 mmol). Le milieu réactionnel est agité pendant 5 minutes, puis est coulée une solution de formiate d'ammonium (11,5 g ; 183 mmol) dans 135 ml de méthanol. Le milieu réactionnel est agité pendant 30 minutes en laissant la température évoluer, puis il est chauffé à 40°C pendant 30 minutes. Le milieu est alors filtré sur lit de Clarcel et est concentré. On coule 40 ml de toluène que l'on évapore ; cette opération est répétée afin d'éliminer le méthanol. Le résidu ainsi obtenu est repris dans 45 ml de THF. On coule ensuite une solution de dicyclohexylcarbodiimide (7,180 g ; 34,5 mmol) dans 20 ml de THF. Le milieu réactionnel est chauffé à 50°C pendant 1 heure. Le mélange est ramené à 20°C puis la dicyclohexylurée est filtrée. Le filtrat est concentré à sec. Le résidu est mis en solution dans 46 ml d'acétonitrile, on lui ajoute 6,0 g (40,5 mmol.) d'iodure de sodium puis 5,13 ml (40,5 mmol) de chlorure de triméthylsilyle. Le milieu réactionnel est maintenu sous agitation à température ambiante pendant 5 heures. On introduit alors 28 ml d'acétonitrile et 5,6 ml d'eau. Le précipité obtenu est filtré puis repris dans 1 ml d'eau, et le pH est ramené à 7,5 par ajout d'une solution d'ammoniaque. Le solide obtenu est filtré et séché. On obtient m = 4,2 g de produit final avec un rendement de 34 % et une proportion énantiomère (+) / énantiomère (-) de 88,4/11,6.

L'analyse HPLC est réalisée sur une colonne Chiralcel OD 25 cm x 4,6 mm, les éluants utilisés sont l'heptane 600 et l'éthanol 400 et le débit est de 1 ml/min 210 nm. Les temps de rétention obtenus sont : - énantiomère (-) : 7,1 minutes
- énantiomère (+) : 9 minutes.

Le produit est repris dans l'acétone (40 ml), puis on ajoute de l'eau (150 ml). On laisse précipiter et on obtient 3 g de produit avec une proportion énantiomère (+) / énantiomère (-) de 99,4 / 0,6.

RMN ¹H (250 MHz, DMSO D6) : 0,8 *(t,* 3H, *CH*_{*3*}-CH₂); 1,65 (*m*, 2H, *CH*_{*2*}-CH₃); 3,00-3,35 (*q*, 1H+1H, -CH₂-C=O); 5,3 (*q*, 2H, CH₂-O); 5,7 (s, -OH); 6,35 *(d,* 1H aromatique); 7,3 *(d,* 1H aromatique); 11,7 *(s,* N-H).

### Exemple 2: (+)-5-éthyl-9,10-difluoro-5-hydroxy-4,5,13,15-tétrahydro-1H,3H-oxépino[3 ',4 ':6, 7]indolizino[1,2-b]quinoline-3,15-dione

### 2.a. N-(3,4-difluorophényl)acétamide

Un mélange de 3,4-difluoroaniline (50 ml; 0,5 mole) et de triéthylamine (70 ml; 0,5 mol) dans du dichlorométhane (1,5 1) est refroidi au moyen d'un bain de glace. De l'anhydride acétique (71,5 ml; 0,75 mol) est ajouté goutte à goutte et le mélange réactionnel est ensuite agité pendant 1 h à température ambiante. Le mélange obtenu est ensuite lavé successivement avec de l'eau, une solution de bicarbonate de sodium à 10 % et de la saumure saturée. La fraction organique est séchée sur du sulfate de sodium et concentrée sous pression réduite. Le résidu est mis en suspension dans du pentane, filtré et séché sous pression réduite pour donner le produit du titre (78 g; 91 % de rendement) sous forme d'un solide blanchâtre (P_{f} 126-127,5 °C).
RMN ¹H (DMSO) : 2,15 (s, 3H); 7,10-7,65 (m, 2H); 7,65-8,10 (m, 1H); 10,30 (pic large, 1H).

### 2.b. 2-chloro-6,7-difluoro-3-quinoline-3-carbaldéhyde:

On a utilisé la procédure générale décrite par Meth-Cohn *et al., J. Chem. Soc. Perkin Trans. I,* **1981**, 1520 et 2509.
A un réactif de Vilsmeyer, obtenu par addition goutte à goutte sous atmosphère d'argon d'oxychlorure de phosphore (103 ml; 1,1 mol) sur du DMF anhydre (34 ml; 440 mmol) refroidi par un bain de glace et agité pendant une demi-heure avant de laisser la température remonter jusqu'à température ambiante, on ajoute le produit de l'étape 2.a (32 g; 220 mmol). Le mélange ainsi obtenu est agité à 70 °C durant 16 h. Après retour à température ambiante, le mélange réactionnel est ajouté goutte à goutte à un mélange eau-glace (400 ml) et agité durant 2 h. Le précipité obtenu est filtré et lavé avec de l'eau, puis séché pour donner le produit du titre (9 g; 18 % de rendement) sous forme d'un solide jaune (P_{f} 226,5-229 °C).
RMN ¹H (DMSO) : 8,17 (dd, 1H); 8,39 (dd, 1H); 8,97 (d, 1H); 10,34 (d, 1H).
IR (KBr): 888, 1061, 1262, 1507, 1691 cm⁻¹.

### 2.c. 2-chloro-6,7-difluoro-3-quinolylméthanol:

Une suspension du produit de l'étape 2.b (9 g; 39 mmol) dans du méthanol (400 ml) est traitée par de l'hydroborure de sodium (2 g; 53 mmol) à température ambiante durant une demi-heure. L'excès de borohydrure est détruit par de l'acide acétique (2 ml). Les substances volatiles sont éliminées sous pression réduite. Le résidu est dissous dans de l'acétate d'éthyle (500 ml), le mélange obtenu étant ensuite lavé successivement avec une solution diluée de bicarbonate de sodium, de l'eau et de la saumure saturée. On sèche ensuite sur du sulfate de magnésium et concentre sous pression réduite. Le résidu est recristallisé dans du 1,2-dichloroéthane pour donner le produit du titre (8 g; 80 % de rendement) sous forme d'un solide beige (P_{f} 166,5-167 °C).
RMN ¹H (DMSO) : 4,67 (d, 2H); 5,80 (t, 1H); 8,01 (dd, 1H); 8,22 (dd, 1H); 8,48 (s, 1H).
IR (KBr) : 871, 1038, 1253, 1513 cm⁻¹.

### 2.d. (+)-8-(2-chloro-6,7-difluoro-3-quinolineméthanol)-5-éthyl-5-hydroxy-1,3,4,5,8,9-hexahydrooxépino[3,4-c]pyridine-3,9-dione:

A une solution dans du DMF anhydre (30 ml) de (+)-**EHHOPD** (1,58 g; 7,08 mmol; étape 1.c.), de produit de l'étape 2.c (1,62 g; 7,06 mmol) et de tributylphosphine (1,91ml; 7,87 mmol), on ajoute goutte à goutte, à température ambiante et sous atmosphère d'argon, du diéthylazodicarboxylate (1,24 ml; 7,87 mmol). Le mélange ainsi obtenu est ensuite agité pendant 16 h. Le milieu réactionnel est ensuite évaporé à sec sous pression réduite. Le résidu est purifié par chromatographie sur colonne de silice (éluant : acétate d'éthyle). Le solide obtenu est repris dans du diéthyléther, filtré et séché pour donner le produit du titre (1,56 g; 51 % de rendement) sous forme d'un solide blanchâtre (P_{f} 196 °C).
RMN ¹H (DMSO) : 0,84 (t, 3H); 1,74 (m, 2H); 3,02 (d, 1H); 3,34 (d, 1H); 5,29 (s, 2H); 5,31 (dd, 2H); 5,75 (s, 1H); 6,51 (d, 1H); 7,80 (d, 1H); 8,03 (dd, 1H); 8,07 (s, 1H); 8,17 (dd, 1H).
IR (KBr) : 875, 1057, 1360, 1507, 1574, 1647, 1749cm⁻¹.

### 2.e. (+)-5-éthyl-9,10-difluoro-5-hydroxy-4,5,13,15-tétrahydro-1H,3H-oxépino [3',4':6,7]indolizino[1,2-b] quinoline-3,15-dione:

Un mélange de produit de l'étape 2.d (1,53 g; 3,52 mmol; étape 2.d.), de bromure de tétrabutylammonium (1,25 g; 3,87 mmol), d'acétate de potassium (520 mg; 5,28 mmol), de triphénylphosphine (180 mg; 0,70 mmol) et d'acétate de palladium (II) (79 mg; 0,35 mmol) est agité sous atmosphère d'argon dans de l'acétonitrile anhydre chauffé à reflux durant 22 h. Après retour du milieu réactionnel à température ambiante, on concentre sous pression réduite avant d'effectuer une chromatographie sur colonne de silice (éluant: mélange CH₂Cl₂/MeOH 98/2). On obtient alors le produit du titre (960 mg; rendement de 68 %; pureté déterminée par HPLC : 97,1 %). Ce produit est repris dans CH₂Cl₂ anhydre (100 ml) et agité 24 h, puis filtré et séché sous pression réduite pour donner le produit du titre purifié (850 mg; rendement de 61 %; pureté déterminée par HPLC : 99,6 %) sous forme d'un solide blanc.
RMN ¹H (DMSO) : 0,87 (t, 3H); 1,85 (m, 2H); 3,08 (d, 1H); 3,44 (d, 1H); 5,26 (s, 2H); 5,39 (d, 2H); 5,52 (d, 2H); 5,99 (pic large, 1H); 7,39 (s, 1H); 8,15 (dd, 1H); 8,23 (dd, 1H); 8,68 (s, 1H).
IR (KBr) : 871, 1261, 1512, 1579, 1654, 1746 cm⁻¹.

### Exemple 3: (+)-chlorure de 1-[9-chloro-5-éthyl-5-hydroxy-10-méthyl-3,15-dioxo-4,5,13,15-tétrahydro-1H,3H-oxépino[3',4':6,7]indolizino[1,2-b]quinolin-12-ylméthyl]-4-méthyl-hexahydropyridinium

### 3.a. 1-(2-amino-4-chloro-5-méthylphényl)-2-chloro-éthanone:

De la 3-chloro-4-méthylaniline (44,4 ml; 0,366 mol) dans du 1,2-dichloroéthane (440 ml), sous atmosphère d'argon, est refroidi par un bain de glace. On ajoute goutte à goutte et dans l'ordre à ce mélange : du trichlorure de bore (1M dans l'heptane; 400 ml; 0,4 mol), du chloroacétonitrile (28 ml; 0,44 mol) et du chlorure de diéthylaluminium (1M dans l'heptane; 400 ml; 0,4 mol). Durant l'addition, la température est maintenue en dessous de 20 °C. Le mélange résultant est ensuite chauffé à reflux durant 3 h, puis refroidi à 10 °C. On procède alors avec précaution à l'hydrolyse du milieu réactionnel au moyen d'acide chlorhydrique 2N (240 ml) et on chauffe à reflux durant 1 h. On ajoute de l'eau (1 l) et de l'acétate d'éthyle (1 l), on agite le mélange obtenu durant 15 mn avant de séparer les phases. La phase aqueuse est à nouveau extraite avec de l'acétate d'éthyle (200 ml), et on lave avec de l'eau (500 ml) les phases organiques combinées. On sèche sur sulfate de magnésium et on concentre la phase organique. Le résidu est repris dans de l'éther de pétrole (fraction ayant un point d'ébullition de 45 à 60 °C; 150 ml) et le mélange ainsi obtenu est laissé reposer durant 16 h à 4 °C. Le précipité résultant est récupéré par filtration, lavé avec de l'éther de pétrole et séché sous pression réduite pour donner le produit du titre (25 g ; 31 % de rendement). P_{f} 129-130 °C.
RMN ¹H (DMSO) : 2,20 (s, 3H); 4,98 (s, 2H); 6,90 (s, 1H); 7,15 (pic large, 2H); 7,70 (s, 1H).
IR (KBr) : 871, 1018, 1183, 1225, 1270, 1533, 1577, 1619, 1662 cm⁻¹.

### 3.b. 7-chloro-4-chlorométhyl-6-méthyl-2-oxo-1,2-dihydro-3-quinolinecarboxylate d'éthyle:

Du produit de l'étape 3.a (25 g; 0,11 mol) et de la triéthylamine (30,6 ml; 0,22 mol) sont mélangés dans de l'acétonitrile (520 ml). Du chlorure d'éthylmalonyle (28,1 ml; 0,22 mol) est ajouté à température ambiante et sous atmosphère d'argon. Le mélange obtenu est agité durant 3 h. De l'éthanolate de sodium (préparé par dissolution de 3 g, soit 0,13 mol, de sodium dans 140 ml d'éthanol absolu) est ensuite ajouté goutte à goutte et le mélange résultant est agité à température ambiante durant 16 h. Le précipité est récupéré par filtration, lavé successivement par de l'éthanol, de l'eau, de l'éthanol et de l'éther. Il est ensuite séché sous pression réduite à 70 °C sur du pentoxide de phosphore pour donner le produit du titre (28,6 g; 83 % de rendement) sous forme d'une poudre blanchâtre.
RMN ¹H (DMSO): 1,30 (t, 3H); 2,40 (s, 3H); 4,35 (q, 2H); 4,85 (s, 2H); 7,41 (s, 1H); 7,91 (s, 1H); 12,15 (pic large, 1H).
IR (KBr) : 879, 1108, 1250, 1288, 1483, 1664, 1721 cm⁻¹.

### 3.c. 2,7-dichloro-4-chlorométhyl-6-méthyl-3-quinolinecarboxylate d'éthyle :

Du produit de l'étape 3.b (28,4 g; 90 mmol) est chauffé durant 4 h à reflux dans de l'oxychlorure de phosphore (400 ml). Le mélange obtenu est concentré sous pression réduite (20 mm Hg) à 80 °C. Le résidu est repris dans du diisopropyléther (400 ml). Le précipité résultant est récupéré par filtration, lavé avec de l'éther et de l'éther de pétrole, puis séché sous pression réduite pour donner le produit du titre (25,4 g; 85 % de rendement) sous forme d'une poudre blanchâtre (P_{f} 126-127 °C).
RMN ¹H (DMSO) : 1,37 (t, 3H); 2,58 (s, 3H); 4,49 (q, 2H); 5,14 (s, 2H); 8,16 (s, 1H); 8,35 (s, 1H).
IR (KBr) : 874, 1006, 1163, 1243, 1278, 1577, 1723 cm⁻¹.

### 3.d. 2,7-dichloro-4-chlorométhyl-6-méthyl-3-quinolylméthanol:

Du produit de l'étape 3.c (25,2 g; 76,5 mmol) est mélangé sous atmosphère d'argon à du dichloroéthane (630 ml). De l'hydrure de diisobutylaluminium (1M dans du dichlorométhane; 307 ml; 307 mmol) est ajouté goutte à goutte tandis que le mélange réactionnel est agité et la température maintenue en dessous de 20 °C. Le mélange réactionnel est ensuite agité à température ambiante pendant 3 heures, puis versé dans une solution aqueuse de tartrate de potassium (concentrée à 20 % en poids; 1,5 1). L'émulsion ainsi obtenue est agitée vigoureusement durant 1 h, filtrée sur célite et les deux phases sont alors séparées. La phase aqueuse est extraite par de l'acétate d'éthyle (200 ml) et les phases organiques combinées sont lavées avec une solution aqueuse de chlorure de sodium (concentrée à 20 % en poids; 500 ml). La phase organique obtenue est séchée sur sulfate de magnésium, filtrée et concentrée sous pression réduite. Le résidu est repris dans du diéthyléther (50 ml) et le précipité résultant est récupéré par filtration. Par séchage sous pression réduite, on obtient le produit du titre (18,3 g; 93 % de rendement) sous forme d'une poudre blanchâtre (P_{f} 169-170 °C).
RMN ¹H (DMSO) : 2,57 (t, 3H); 4,84 (s, 2H); 5,36 (s, 2H); 8,06 (s, 1H); 8,27 (s, 1H).
IR (KBr): 870, 1022, 1102, 1304, 1482, 1567 cm⁻¹.

### 3.e. 2,7-dichloro-6-méthyl-4-(4-méthylpipéridinométhyl)-3-quinolylméthanol:

Une solution de produit de l'étape 3.d (16,2 g; 55,7 mmol) dans du THF (70 ml) est traitée par une solution de 4-méthylpipéridine (23 ml; 195 mmol). Le mélange obtenu est agité à température ambiante durant 2 h. On ajoute de l'eau (200 ml) et du dichloroéthane (200 ml). La phase organique est lavée avec une solution aqueuse de chlorure de sodium (concentrée à 20 % en poids; 100 ml), séchée sur du sulfate de magnésium et concentrée sous pression réduite. Par cristallisation du résidu dans du diéthyléther, on obtient le produit du titre (18,3 g; 93 % de rendement) sous forme d'un solide cristallin blanc (P_{f} 170-171,5 °C).
RMN ¹H (CDCl₃): 0,88 (d, 3H); 1,17 (m, 2H); 1,42 (m, 1H); 1,60 (m, 2H); 2,19 (t, 2H); 2,56 (s, 3H); 2,82 (d, 2H); 4,02 (s, 2H); 4,93 (s, 2H); 6,36 (pic large, 1H); 7,95 (s, 1H); 8,02 (s, 1H).
IR (KBr) : 971, 1013, 1105, 1293, 1479, 1559 cm⁻¹.

### 3.f. (+)-8-[2,7-dichloro-6-méthyl-4-(4-méthylpipéridinométhyl)-3-quinolylméthyl]-5-éthyl-5-hydroxy-1,3,4,5,8,9-hexahydrooxépino[3,4-c]pydidine-3,9-dione:

Une suspension de (+)**-EHHOPD** (obtenue à l'étape 1.c.; 1,56 g; 7,0 mmol) dans du dioxane anhydre (70 ml) est traitée successivement, sous atmosphère d'argon, par du produit de l'étape 3.e (2,47 g; 7,0 mmol), de la triphénylphosphine (2,02 g; 7,7 mmol) et du diisopropyl azodicarboxylate (1,07 ml; 10,5 mmol). Le mélange est agité à température ambiante durant 16 h. Les substances volatiles sont ensuite évaporées sous pression réduite. Le résidu est purifié par chromatographie sur colonne de silice (éluant : acétate d'éthyle). Le solide obtenu est repris dans du diéthyléther, filtré et séché pour donner le produit du titre (1,96 g; 50 % de rendement) sous forme d'un solide blanchâtre (P_{f} 182°C).
RMN ¹H (DMSO) : 0,89 (m, 8H); 1,23 (m, 1H); 1,41 (t, 2H); 1,64 (m, 2H); 2,09 (q, 2H); 2,59 (m, 5H); 3,15 (dd, 2H); 4,06 (dd, 2H); 5,31 (dd, 2H); 5,35 (dd, 2H); 5,75 (s, 1H); 6,29 (d, 1H); 7,17 (d, 1H); 8,06 (s, 1H); 8,46 (s, 1H).
IR (KBr) : 878, 1053, 1275, 1474, 1572, 1648, 1747 cm⁻¹.

### 3.g. (+)-9-chloro-5-éthyl-5-hydroxy-10-méthyl-12-(4-méthylpipéridinométhyl)-4,5,13,15-tétrahydro-1H,3H-oxépino[3',4' :6,7]indohzino[1,2-c]quinoline-3,15-dione

Un mélange de produit de l'étape 3.f (3,80 g; 6,80 mmol), de bromure de tétrabutylammonium (2,42 g; 7,5 mmol), d'acétate de potassium (1,00 g; 10,2 mmol), de triphénylphosphine (890 mg; 3,4 mmol) et d'acétate de palladium (II) (220 mg; 0,68 mmol) est agité sous atmosphère d'argon dans de l'acétonitrile anhydre (85 mg) à reflux durant 24 h. Après refroidissement à température ambiante, le précipité résultant est récupéré par filtration et lavé successivement par de l'acétonitrile, de l'eau, de l'acétone et du diéthyléther pour donner, après séchage sous pression réduite, le produit du titre (2,5 g; 70 % de rendement) sous forme d'une poudre blanchâtre.
RMN ¹H (DMSO): 0,86 (m, 6H); 1,12 (q, 2H); 1,36 (m, 1H); 1,56 (d, 2H); 1,84 (q, 2H); 2,12 (t, 2H); 2,56 (s, 3H); 2,83 (dd, 2H); 3,26 (dd, 2H); 4,03 (dd, 2H); 5,28 (dd, 2H); 5,45 (dd, 2H); 6,04 (s, 1H); 7,34 (s, 1H); 8,14 (s, 1H); 8,38 (s, 1H).
IR (KBr): 870, 1058, 1208, 1280, 1477, 1593, 1655, 1749 cm⁻¹.

### 3.h. (+)-chlorure de 1-[(5R)-9-chloro-5-éthyl-5-hydroxy-10-méthyl-3,15-dioxo-4,5,13,15-tétrahydro-1H,3H-oxépino[3',4':6,7]indolizino[1,2-c]quinolin-12-ylméthyl]-4-méthyl-hexahydropyridinium:

Un mélange de produit de l'étape 3.g (2,3 g; 7,7 mmol) et d'éthanol absolu (300 ml) est soumis pendant 2 minutes à des ultrasons. La suspension laiteuse obtenue est agitée et traitée par de l'acide chlorhydrique (solution 1N; 13,2 ml; 13,2 mmol) pour donner une solution jaune clair qui, au repos, forme un précipité de type gel. Le précipité est récupéré par filtration sur Büchner et lavé successivement avec de l'éthanol et de l'éther, puis séché sous pression réduite pour donner le produit du titre (2,1 g; 85 % de rendement).
RMN ¹H (DMSO) : 0,87 (m, 6H); 1,59 (m, 5H); 1,84 (q, 2H); 2,64 (s, 3H); 3,28 (dd, 2H); 3,45 (s, 2H); 4,93 (s, 2H); 5,47 (dd, 2H); 5,61 (s, 2H); 6,04 (pic large, 1H); 7,41 (s, 1H); 8,28 (s, 1H); 8,63 (s, 1H); 10,30 (pic large, 1H).
IR (KBr) : 1043, 1212, 1479, 1585, 1655, 1751 cm⁻¹.

### ÉTUDE PHARMACOLOGIQUE DES PRODUITS DE L'INVENTION

### Test sur la prolifération cellulaire.

Cinq lignées de cellules tumorales sont utilisées dans cette étude : SW620 (adénocarcinome de colon humain), OVCAR-5 (adénocarcinome d'ovaire humain), PC-3 et DU 145 (lignée cellulaire de prostate humaine) et NCI-H69 (adénocarcinome de poumon humain). Ces lignées proviennent du NCI/Frederick Cancer Research and Development Center (Frederick, MD). Elles sont cultivées en milieu complet comprenant le milieu RMPI-1640 enrichi de 10 % de sérum de veau foetal et de 2 mM de L-Glutamine. Elles sont incubées à 37°C en atmosphère humide à 5 % de CO₂. Les cellules adhérentes sont décollées par un traitement avec une solution à 0,25 % de trypsine et 0,2% d'EDTA (Worthington Biochemical Corp., Freehold, NJ) pendant 5 minutes à 37 °C. Le comptage des cellules se fait à l'aide d'un compteur Coulter Z1 (Coulter Corp., Hialeah, FL). La viabilité est évaluée en colorant les cellules avec de l'iodure de propidium puis en les dénombrant avec un cytomètre en flux EPICS Elite (Coulter).

Le composé des exemples 2 et 3 à tester est dissout à 5 mM dans une solution de N,N-diméthylacétamine (DMA, Aldrich). Les dilutions ultérieures sont effectuées avec du milieu de culture. Les concentrations molaires finales testées sont : 1.10⁻⁶, 2.10⁻⁷, 4.10⁻⁸, 8.10⁻⁹, 1,6.10⁻⁹, 3,2.10⁻¹⁰, 6,4.10⁻¹¹, 1,28.10⁻¹¹, 2,56.10⁻¹², et 5,12.10⁻¹³. Chaque concentration est testée sur huit puits. Des contrôles sur l'influence du DMA ont été effectués sur toutes les lignées cellulaires. Il résulte de ces contrôles qu'à la concentration maximale utilisée (0,02 %) le DMA n'a pas d'effet. La doxorubicine aux concentrations de 1.10⁻⁷ M et 2.10⁻⁷ M est utilisée comme contrôle positif.

Les cellules sont ensemencées à 5.10³ cellules par puit sur une microplaque à 96 puits (Costar Corporation, Cambridge, MA). Les cellules sont incubées 24 h à 37 °C afin de permettre une reprise de la multiplication cellulaire. Le composé des exemples 2 et 3 à tester est ensuite ajouté aux concentrations indiquées ci-dessus et les cellules sont incubées à 37 °C en atmosphère humide à 5 % de CO₂, pendant 3 jours pour les cellules adhérentes (SW620, OVCAR-5, PC-3 et DU 145) et pendant 5 jours pour les cellules en suspension (NCI-H69).

Les cellules adhérentes sont testées par la méthode SRB (décrite par L.V. Rubenstein, R.H. Shoemaker, K.D. Paull, R.M. Simon, S. Tosini, P. Skehan, D.A Scudiero., A. Monks, and M.R. Boyd "Comparison of in vitro anticancer-drug-screening data generated with tetrazolium assay versus a protein assay against a diverse panel of human tumor cell lines", *J*. *Nat. Cancer Inst.,* **82**:1113-1118, 1990). Après trois jours d'incubation le surnageant est éliminé et 200 µl RPMI-1640 exempt de sérum de veau foetal sont ajoutés. Les cellules sont fixées par addition de 50 µl d'acide trichloroacétique à 50 % (concentration finale d'acide trichloroacétique de 10 %) et incubées à 4 °C pendant 1 heure. Les puits sont lavés 5 fois avec de l'eau puis colorés par 50 µl d'une solution à 0,4 % de sulforhodamine B (SRB, Sigma) dans de l'acide acétique à 1 % à température ambiante pendant 10 minutes. La teinture est solubilisée avec 100 µl de tampon TRIS à 10 mM, pH 10, pendant environ 5 minutes avec agitation, les microplaques sont lues par spectrophotométrie à 570 nm.

Les cellules en suspension sont testées par la méthode XTT (décrite par D.A. Scudiero, R.H. Shoemaker, K.D. Paull, A. Monks, S. Tierney, T.H. Nofziger, M.J. Currens, D. Seniff et M.R. Boyd : "Evaluation of a soluble tetrazolium/formazan assay for cell growth and drug sensitivity in culture using human and other tumor cell lines", *Cancer Research* **48**:4827-4833, 1988). Après incubation en présence du composé des exemples 2 et 3 à tester, le XTT [sel sodique du 2,3-bis(2-méthoxy-4-nitro-5-sulfophényl)-2H-tétrazolium-5-carboxanilide, (Sigma)] et le méthosulfate de phénazine (PMS, Sigma) en solution dans du tampon phosphate salin sont ajoutés aux cultures, et les cellules sont incubées pendant 4 heures à 37°C dans une atmosphère à 5 % de CO₂. Les concentrations finales de XTT et de PMS sont respectivement de 50 et 0,38 µg/puit. La production de formazan est stoppée par addition de 10 µl de dodécylsulfate de sodium à 10 % (Sigma) et l'absorbance est lue par spectrophotométrie à 450 nm avec un filtre de référence à 600-650 nm.

### Résultats

Les concentrations molaires des composés des exemples 2 et 3 inhibant de 50% la prolifération cellulaire sont compilées dans le tableau suivant :

| **Lignée cellulaire** | **Exemple 2** | **Exemple 3** |
|---|---|---|
| **SW620** | 5.10⁻⁹ | 3.10⁻⁸ |
| **OVCAR-5** | 8.10⁻⁹ | 4.10⁻⁸ |
| **PC-3** | 1.10⁻⁸ | 3.10⁻⁸ |
| **DU 145** | 1.10⁻⁹ | 7.10⁻⁹ |
| **NCI-H69** | 3.10⁻¹⁰ | 1.10⁻⁹ |

## Revendications

1. Produit de formule (**I**) représentée ci-dessous

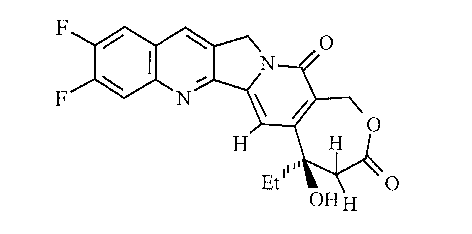

2. A titre de médicament, un produit selon la revendication 1.

3. Composition pharmaceutique contenant, à titre de principe actif, au moins le composé de la revendication 2.

4. Utilisation du composé selon la revendication 1 pour la préparation de médicaments antitumoraux.

5. Utilisation du composé selon la revendication 1 pour la préparation de médicaments antiviraux.

6. Utilisation du composé selon la revendication 1 pour la préparation de médicaments antiparasitaires.
